Europäisches Patentamt

European Patent Office

Office européen des brevets

(11) Veröffentlichungsnummer: **0 372 501**
**A2**

## (12) EUROPÄISCHE PATENTANMELDUNG

(21) Anmeldenummer: 89122438.8

(22) Anmeldetag: 05.12.89

(51) Int. Cl.⁵: **A61K 39/39, A61K 39/095,**
**C07K 7/00, C07K 3/06,**
**A61K 39/395, G01N 33/68**

(30) Priorität: 07.12.88 DE 3841091

(43) Veröffentlichungstag der Anmeldung:
**13.06.90 Patentblatt 90/24**

(84) Benannte Vertragsstaaten:
**AT BE CH DE ES FR GB GR IT LI LU NL SE**

(71) Anmelder: **BEHRINGWERKE**
**Aktiengesellschaft**
**Postfach 1140**
**D-3550 Marburg 1(DE)**

(72) Erfinder: **Pelzer, Hermann, Dr.**
**Calvinstrasse 3**
**D-3550 Marburg(DE)**
Erfinder: **Stüber, Werner, Dr.**
**Cölber Weg 12**
**D-3551 Lahntal(DE)**
Erfinder: **Hungerer, Klaus-Dieter, Dr.**
**Oberer Eichweg 16**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Synthetische Antigene, Verfahren zu ihrer Herstellung und ihre Verwendung.**

(57) Es werden Konjugate aus Membranproteinen von Neisseria meningitidis und Peptiden beschrieben, die als Arzneimittel verwendet werden können.

Sie sind weiterhin zur Herstellung von Antikörpern geeignet, die zur Therapie und Diagnose verwendet werden können.

EP 0 372 501 A2

## Synthetische Antigene, Verfahren zu ihrer Herstellung und ihre Verwendung

Die Erfindung betrifft synthetische Antigene, die aus einem Peptid und einem Membranprotein aus Neisseria meningitidis als antigenisierendem Molekül bestehen, ein Verfahren zu deren Herstellung, ihre Verwendung zur Gewinnung von Antikörpern sowie die Verwendung der Antigene oder Antikörper als Arzneimittel und der Antikörper für diagnostische Zwecke.

In EP-A-0 161 188 ist beschrieben, daß Membranproteine aus Neisseria meningitidis an Polysaccharide gebunden werden können, um deren Antigenität zu verbessern.

Nach dem Stand der Technik werden Peptide, die nicht antigen sind (Haptene), und gegen die ein Antikörper erzeugt werden soll, an ein hochmolekulares, meist lösliches Molekül gebunden und sogenannte Konjugate erzeugt und dann Tiere, beispielsweise Kaninchen oder Mäuse damit immunisiert. Nach einer Verweilzeit von einigen Tagen oder Wochen, werden dem Tier nach eventuellen Boosterschritten Blutproben entnommen und die Antikörper gewonnen.

Als hochmolekulare Moleküle werden meist Proteine benutzt, wobei das Keyhole Limpet Hemocyanin und Albumine besonders zu nennen sind.

Keyhole Limpet Hemocyanin-Konjugate besitzen die Nachteile, daß sie schwer löslich sind, und daß Antikörper gegen Keyhole Limpet Hemocyanin induziert werden.

Diese Nachteile wurden nun, überraschenderweise, durch die vorliegende Erfindung behoben, indem als antigenisierendes Protein für nicht antigene Peptide, vorzugsweise Oligo-oder Polypeptide mit bis zu 100 Aminosäuren, ein Membranprotein aus Neisseria meningitidis eingesetzt wird.

Gegenstand der Erfindung sind daher Konjugate aus einem Membranprotein aus Neisseria meningitidis und einem Peptid.

Vorzugsweise wird das Meningokokken-Protein $B_2$ verwendet.

Die Herstellung der Peptid-Protein-Konjugate erfolgt nach an sich bekannten Methoden, beispielsweise durch Kopplung über Glutardialdehyd oder durch Einführung von Maleimidogruppen am Protein mit nachfolgender Bindung eines Peptids über dessen freie Sulfhydrylfunktion.

Zur Herstellung der erfindungsgemäßen Peptid-Protein-Konjugate mittels Glutardialdehyd werden beispielsweise das Meningokokken B-Protein in einem Puffer gelöst, vorzugsweise 1 mg Protein in 1 ml PBS (phosphate buffered saline),das Peptid zugesetzt, vorzugsweise 1 - 2 mg Peptid pro mg Protein, und pro mg Protein 5 - 20 µl, bevorzugt 10 µl, einer 25%igen wässrigen Lösung von Glutardialdehyd zugegeben. Die Lösung wird bei Raumtemperatur 30 min bis 5 Stunden, vorzugsweise 1 Stunde, gerührt, und zur Blockierung reaktiver Aldehydgruppen werden pro mg Protein 40 mg Lysin zugesetzt. Nach Ablauf weiterer 10 min wird gegen destilliertes Wasser dialysiert und das Konjugat vorzugsweise durch Lyophilisation gewonnen.

Zur Bindung von Thiolgruppen enthaltenden Peptiden an das Protein können Thiolgruppen über Disulfidbrücken oder Thioether an das Meningokokken-B-Protein gebunden werden, wobei die Konjugatherstellung vorzugsweise über Thioetherbindung stattfindet.

In einer besonders bevorzugten Ausführung der Konjugierung wird zunächst das Protein derartig derivatisiert, daß dann Thiolgruppen direkt an das Protein gebunden werden können. Hierzu wird das Protein in einem Puffer pH 7 - 9, vorzugsweise einem Natriumhydrogencarbonatoder -phosphatpuffer, gelöst und mit einer N-Maleimidogruppen enthaltenden Substanz, vorzugsweise N-Maleimidobuttersäurehydroxysuccinimidester, umgesetzt. Nach Abtrennung des so "aktivierten" Proteins, beispielsweise durch Gelpermeation an Sephadex[R]-G 50 gibt man pro mg Protein 1 - 3 mg Thiolgruppen enthaltendes Peptid hinzu und läßt den Ansatz 0.5 - 10 Stunden, vorzugsweise 1 Stunde, reagieren. Das Protein-Peptidkonjugat kann dann wie üblich, vorzugsweise durch Dialyse und Lyophilisation, gewonnen werden.

Mit einem so hergestellten Konjugat erfolgt die Immunisierung geeigneter Tiere, wobei Antikörper gegen das synthetische Peptid gebildet werden. Bevorzugte Tierspezies für Immunisierung und Antikörpergewinnung ist hierbei das Kaninchen. Das synthetische Antigen kann ebenso zur Immunisierung von Mäusen und unter Anwendung der Hybridoma-Technik zur Herstellung monoklonaler Antikörper verwendet werden.

Das durch Immunisieren mit dem Konjugat erhaltene Antiserum reagiert spezifisch mit dem Protein, aus welchem die zur Immunisierung verwendete Peptidsequenz stammt.

Neben der guten Löslichkeit des Konjugats hat sich überraschenderweise gezeigt, daß das gegen das Peptid gerichtete Antiserum in der Doppelimmundiffusion überwiegend keine Reaktivität mit dem Meningokokkenprotein gezeigt. Dies ist umso überraschender, als bei Verwendung anderer antigenisierender Proteine stets auch Antikörper gegen diese induziert werden.

Die erfindungsgemäß gewonnenen Antikörper können für eine Reihe verschiedenartig konzipierter Immunoassays eingesetzt werden.

Die Beispiele erläutern die Erfindung. In den Beispielen werden folgende Abkürzungen verwendet:

GMBS gamma-Maleimidobuttersäure-N-hydroxysuccinimidester

Cys L-Cystein

Leu L-Leucin

Phe L-Phenylalanin

Ser L-Serin

Pro L-Prolin

Glu L-Glutaminsäure

Lys L-Lysin

Gly Glycin

Val L-Valin

Ala L-Alanin

Arg L-Arginin

Asp L-Asparaginsäure

Tyr L-Tyrosin

Asn L-Asparagin

KLH Keyhole Limpet Hämocyanin

AT III Antithrombin III


Beispiele


Beispiel 1


a) Derivatisierung von Meningokokken-B-Protein mit Maleimidogruppen

40 mg Meningokokken-B-Protein wurden in 0.05 mM Natriumphosphatpuffer pH 8.0 gelöst und mit 5 mg GMBS 1 Stunde lang aktiviert. Das Rohprodukt wurde an einer Sephadex$^{(R)}$-G 50-Säule (2 x 30 cm) in 0.1 M Natriumphosphat/0.5 mM EDTA pH 6.0 chromatographiert. Die Proteinfraktion wird gesammelt und auf ca. 8 ml konzentriert.


b) Herstellung des Peptid-Protein-Konjugates Meningokokken-B-Protein-(AT III 343-363)

Zu der Proteinfraktion (siehe Beispiel 1a) wurden 40 mg Peptid der Struktur Cys-Leu-Phe-Ser-Pro-Glu-Lys-Ser-Lys-Leu-Pro-Gly-Ile-Val-Ala-Ala-Glu-Gly-Arg-Asp-Asp-Leu-Tyr (AT III 343 - 363), das mittels der Festphasen-Peptid-Methode hergestellt wurde, gegeben und der Ansatz 1 Stunde unter Ausschluß von Sauerstoff gerührt. Nach Dialyse und Lyophilisation wurden 48 mg Peptid-Protein-Konjugat erhalten.


Beispiel 2


a) Derivatisierung von Keyhole-Limpet-Hämocyanin mit Maleimidogruppen

In Analogie zu Beispiel 1a) wurden 40 mg KLH derivatisiert.


b) Herstellung von KLH-(AT III 343 - 363)

In Analogie zu Beispiel 1b) wurde 40 mg AT III 343-363-Peptid an nach Beispiel 2a) derivatisiertes KLH gekoppelt. Ausbeute 45 mg.


Beispiel 3

Herstellung von Meningokokken-B-Protein-(AT III 129 -140)

40 mg des Peptids Cys-Arg-Leu-Tyr-Arg-Lys-Ala-Asn-Lys-Ser-Ser-Lys-Leu (AT III 129 - 140) wurden nach Beispiel 1 an "aktiviertes" Meningokokken-B-Protein gekoppelt. Ausbeute 47 mg.

Beispiel 4

Herstellung von KLH-(AT III 129 - 140)

In Analogie zu Beispiel 3 wurde das AT III 129-140-Peptid an GMBS derivatisiertes KLH gekoppelt. Ausbeute 49 mg.

Beispiel 5

Immunisierung von Kaninchen und Austestung der Antisera

5 Kaninchen wurden mit jeweils 2 mg Antigen über einen Zeitraum von 8 Wochen immunisiert, die Applikation der Konjugate erfolgte subcutan und intravenös. Danach wurden die Tiere entblutet und die gewonnenen Antisera gepoolt und mit Konservierungsmitteln stabilisiert.

Beispiel 6

a) Testung der Antisera mittels Enzymimmunoassay-Technik (ELISA)

Die Testung der Immunreaktion der gewonnenen Antisera erfolgte mittels ELISA-Technik. Hochgereinigtes AT III-Antigen wurde mit Tris-Pufferlösung (0.025 mol/l) auf eine Konzentration von 10 $\mu$g/ml verdünnt und durch Adsorption an Polystyrol-Röhrchen immobilisiert. Das zu testende Antiserum wurde mit Inkubationspuffer (0.01 mol/l Tris, 0.05 % Tween, pH 7.6) 1 + 1 verdünnt und jeweils 200 $\mu$l pro Röhrchen 30 min bei 37°C inkubiert. Anschließend wurde die Inkubationslösung entfernt und das Röhrchen zweimal mit je 500 $\mu$l Waschlösung (0.02 mol/l Natriumphosphat, 0.05 % Tween, pH 7.6) gewaschen. Danach wurden 200 $\mu$l Peroxidase-konjugierter Anti-Kaninchengammaglobulin-Antikörper zugefügt und die Röhrchen 30 min bei 37°C inkubiert. Nach Entfernen der Konjugat-Lösung und zweimaligem Waschen wurden 200 $\mu$l Substrat-Chromogen-Lösung (Wasserstoffperoxid, o-Phenylendiamin) zugefügt und die Röhrchen bei 18 - 22°C inkubiert. Nach 30 min wurde die Peroxidase mit Schwefelsäure inaktiviert und die Absorption der Reaktionslösung bei 492 nm bestimmt.

Die ELISA-Technik wurde zur Testung aller Antisera angewandt, die unter Verwendung der unter Punkt 1b), 2b), 3) und 4) beschriebenen Peptid-Protein-Konjugate erhalten wurden. Dabei wurden die Antisera in unter-schiedlichen Verdünnungen eingesetzt (1:$10^1$-1:$10^5$-Verdünnung).

In der nachfolgenden Tabelle sind die Absorptionswerte bei 492 nm in Abhängigkeit von der Antiserumverdünnung zu der Absorption eines Röhrchens mit Pufferlösung dargestellt.

Die Ähnlichkeit der gemessenen Absorptionswerte der jeweiligen Verdünnungsstufen belegt, daß sich sowohl KLH als auch Meningokokkenprotein gleich gut als hochmolekulares antigenisierendes Protein eignen, wobei jedoch das mit Meningokokkenprotein-Konjugat gewonnene Antiserum in der Doppelimmundiffusion mit Meningokokkenprotein nicht reagierte (siehe b)).

Tabelle 1

| Antiserum gegen | Verdünnungsstufe | OD 492 nm/30 min |
|---|---|---|
| Peptid-konjugiertes Meningokokken-B-- Protein (AT III 343 - 363) | 1 : 10<br>1 : 100<br><br>1 : 1000<br>1 : 10000<br>1 : 100000 | 2.0<br>1.68 .<br><br>1.30<br>0.77<br>0.63 |
| Peptid-konjugiertes Meningokokken-B-- Protein (AT III 129-140) | 1 : 10<br>1 : 100<br><br>1 : 1000<br>1 : 10000 | 1.28<br>0.83<br><br>0.26<br>0.09 |
| Peptid-konjugiertes KLH (AT III 343-363) | 1 : 10<br>1 : 100<br>1 : 1000<br>1 : 10000<br>1 : 100000 | 2.0<br>1.76<br>1.32<br>0.79<br>0.72 |
| Peptid-konjugiertes KLH-(AT III 129-140) | 1 : 10<br>1 : 100<br>1 : 1000<br>1 : 10000 | 1.03<br>0.84<br>0.34<br>0.14 |
| Puffer-Leerwert | | 0.03 |

b) Testung der Antisera in der Doppelimmundiffusions-Technik

Die unter Punkt 6a) beschriebenen Antisera wurden in der Doppelimmundiffusionstechnik auf Präzipitat-Bildung gegenüber dem Trägerprotein untersucht.

Die mit KLH-Peptid-Konjugat erhaltenen Antisera zeigten in Verdünnungen bis 1 : 16 gegenüber dem KLH-Antigen eine deutliche Präzipitat-Linie im Agarose-Gel. Demgegenüber ergaben die mit Meningokokken-Peptid-Konjugat erhaltenen Antisera mit Meningokokken-B-Protein keine Immunpräzipitate.

**Ansprüche**

1. Konjugate aus einem Membranprotein aus Neisseria meningitidis und einem Peptid.

2. Konjugat nach Anspruch 1, dadurch gekennzeichnet, daß das Protein das Meningokokken-Protein $B_2$ ist.

3. Konjugat nach Anspruch 1, dadurch gekennzeichnet, daß das Peptid aus bis zu 100 Aminosäuren aufgebaut ist.

4. Konjugat nach Anspruch 1, dadurch gekennzeichnet, daß das Peptid ein Peptid aus Antithrombin III ist.

5. Konjugat nach Anspruch 1 als Arzneimittel.

6. Verfahren zur Herstellung eines Konjugats aus einem Membranprotein aus Neisseria meningitidis und einem Peptid, dadurch gekennzeichnet, daß die Bindung zwischen dem Protein und dem Peptid mittels eines peptidchemischen Verfahrens hergestellt wird.

7. Verwendung eines Konjugats nach Anspruch 1 zur Herstellung von Antikörpern.

8. Verwendung eines Antikörpers nach Anspruch 7 als Arzneimittel oder Diagnostikum.

Patentansprüche für folgende Vertragsstaaten: ES, GR

1. Verfahren zur Herstellung eines Konjugats aus einem Membranprotein aus Neisseria meningitidis und einem Peptid, dadurch gekennzeichnet, daß die Bindung zwischen dem Protein und dem Peptid mittels eines peptidchemischen Verfahrens hergestellt wird.

2. Verfahren nach Anspruch 1, dadurch gekennzeichnet, daß das Protein das Meningokokken-Protein $B_2$ ist.

3. Verfahren nach Anspruch 1, dadurch gekennzeichnet, daß das Peptid aus bis zu 100 Aminosäuren aufgebaut ist.

4. Verfahren nach Anspruch 1, dadurch gekennzeichnet, daß das Peptid ein Peptid aus Antithrombin III ist.